# EUROPEAN PATENT APPLICATION

(11) **EP 3 636 754 A1**
(43) Date of publication of application: **15.04.2020**
(21) Application number: 18813909.1
(22) Date of filing: 07.06.2018
(51) Int. Cl.: C12N 15/09, A61K 31/7105, A61K 31/711, A61K 48/00, A61P 27/02, A61P 43/00, C12N 9/16, C12N 15/113

(54) **GENE THERAPY FOR GRANULAR CORNEAL DYSTROPHY**

(30) Priority: 07.06.2017 JP 2017112406
(71) Applicant: The University Of Tokyo, Tokyo 113-8654 (JP); National University Corporation Chiba University, Chiba-shi, Chiba 263-8522 (JP)
(72) Inventor: USUI, Tomohiko, Tokyo 113-8654 (JP); TAKETANI, Yukako, Tokyo 113-8654 (JP); OUCHI, Yasuo, Chiba-shi Chiba 260-8670 (JP)
(74) Representative: Zacco Sweden AB
(86) International application number: PCT/JP2018/021797
(87) International publication number: WO 2018/225807

(57) **Abstract**

The present invention relates to a guide RNA molecule that hybridizes with a target sequence containing a mutation site of a transforming growth factor β-induced (TGFBI) gene associated with granular corneal degeneration.

## Description

### Technical Field

The present invention relates to a gene therapy drug for granular corneal degeneration.

### Background Art

Transparency of the cornea is extremely important for maintaining vision, and clouding of the cornea causes a significant decrease in visual acuity. Various genetic diseases are known to be associated with the cornea. For example, when a person is afflicted with corneal degeneration, a white deposit forms in the cornea and the cornea becomes increasingly opaque with age, gradually resulting in a loss of vision. Among the forms of corneal degeneration, the form of the disease that occurs with the highest incidence among Japanese is granular corneal degeneration. In patients suffering from granular corneal degeneration, the inherently transparent cornea becomes increasingly opaque with age, and vision decreases considerably starting around age 60.

Granular corneal dystrophy (GCD), which is an autosomal dominant genetic disease, is known to be a disease that causes corneal opacity. GCD is caused by a point mutation in the transforming growth factor β-induced (TGFBI) gene located on chromosome 5q31, and results in the formation of multiple irregularly shaped areas of granular opacity in the corneal stroma. Granular corneal dystrophy type 2 (GCD2), which is a type of TGFBI-associated corneal dystrophy, occurs as a result of arginine being replaced with histidine at the 124th amino acid of TGFBI protein. In South Korea, roughly one of 870 persons suffers from GCD2 and the number of those patients is estimated to at least 50,000 persons.

Known methods used to treat diseases that cause corneal opacity consist of corneal transplant and removal of corneal opacity by superficial keratectomy using an argon fluoride gas excimer laser (wavelength: 193 nm). Corneal transplants performed in recent years are broadly divided into two types consisting of penetrating keratoplasty (PKP), in which all five layers of the cornea, consisting of the corneal epithelial layer, Bowman's membrane, corneal stromal layer, Descemet's membrane and corneal endothelial layer, are transplanted, and deep anterior lamellar keratoplasty (DALK), in which the three surface layers are replaced. Both types of procedures have produced favorable transplant results and offer the advantage of enabling an opacity-free state to be maintained for many years. However, these procedures are highly invasive, complications may occur accompanying transplant, and there is also a risk of recurrence.

Keratectomy using an excimer laser also offers various advantages, such as having relatively low invasiveness in comparison with corneal transplants, enabling the surgery to be repeated, and being able to prolong the amount of time until corneal transplant is required. However, refraction may change resulting in farsightedness due to a decrease in the thickness of the cornea. In addition, keratectomy is also associated with post-surgical discomfort and there are also problems with recurrence in the same manner as corneal transplants.

### Citation List

### Non-Patent Document

Non-Patent Document 1: Mannis MJ and Holland EJ, Cornea: fundamentals, diagnosis and management, Fourth edition, volume 1, p.781-785, Elsevier

### Summary

### Technical Problem

At present, a treatment method capable of treating GCD2 has not been established. In addition, corneal transplant and keratectomy using an excimer laser still have many shortcomings requiring improvement as previously described.

With the foregoing in view, an object of the present invention is to solve the above-mentioned problems associated with genetic diseases affecting the cornea such as granular corneal dystrophy, and particularly GCD2, by employing a novel approach that is less invasive than conventional treatment methods while preventing the recurrence of symptoms following treatment.

### Solution to Problem

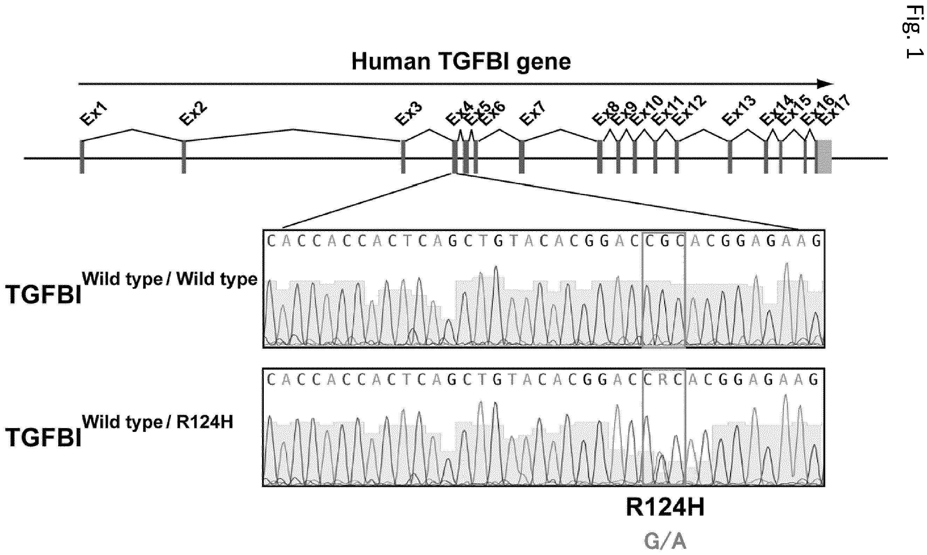

In order to realize radical treatment, it is necessary to repair the mutation in the gene sequence corresponding to the 124th histidine of TGFBI protein to the normal gene sequence (Fig. 1). Although methods using incidentally occurring homologous gene recombination were conventionally carried out focusing primarily on development engineering on embryonic stem cells by introducing a foreign gene containing a homologous sequence on both ends into a gene transfer site in order to carry out genetic modification, this is known to be strongly influenced by the state of the cell's chromatin and the methylated state of the DNA (Feng Liang, et al., "Studies on the influence of cytosine methylation on DNA recombination and end-joining in mammalian cells", J. Biol. Chem. 1995 Oct. 6; 270(40): 23838-44 (http://www.jbc.org/content/270/40/23838.full.pdf)). On the basis thereof, gene transfer utilizing homologous gene recombination is typically extremely difficult in somatic cells following differentiation in which the chromatin structure thereof has become condensed, and in fact, there are no reports describing gene transfer using this technique in keratocytes.

In recent years, the appearance of a novel genetic modification technology referred to as genome editing technology using zinc finger nucleases (ZFN), TALEN or CRISPR/Cas9 and the like has made it possible to easily cleave a target gene sequence. Although regions containing double-strand breaks (DSB) of genomic DNA obtained with these genome editing tools are repaired by either of two types of repair functions consisting of non-homologous end joining (NHEJ) or homology directed repair (HDR), the gene sequence at a cleavage site can be replaced by introducing a template nucleic acid having a sequence homologous to the cleavage site to induce HDR. HDR technology is a revolutionary technology that makes it possible to edit gene sequences as desired by targeting a specific sequence of a genome.

However, in order to realize genome editing technology that is highly efficient, highly specific and has favorable reproducibility, the highly efficient introduction of genome editing tools into cells along with the precise design of those genome editing tools are required.

Although CRISPR/Cas9, which is an RNA-guided genome editing tool, cleaves the both strands of any DNA sequence complementarily bound to guide RNA (gRNA) using a complex of RNA, consisting of about 120 nucleotides and having a sequence required to recognize the target sequence of 20 nucleotides of the gRNA, and SpCas9 protein in the case of using a commonly used RNA-guided nuclease derived from Streptococcus pyogenes in the form of Cas9 (SpCas9), cleavage is not possible unless the three nucleotides of NGG referred to as a protospacer adjacent motif (PAM) sequence are present immediately after the 20-nucleotide target sequence.

In addition, since double-strand cleavage by SpCas9 occurs 3 to 4 nucleotides upstream from this PAM sequence, in the case the desired cleavage site has been determined, double-strand cleavage of the site using SpCas9 is not possible unless the PAM sequence is downstream therefrom. Moreover, a higher GC content is known to result in favorable cleavage efficiency within a range of the GC content in the gRNA nucleotide sequence of 40% to 80%. In addition, non-specific cleavage due to off-target effects has also been reported, and since there is the possibility of a sequence containing three base mismatches with respect to a target sequence being cleaved, it is necessary to reduce the number of sequences resulting in mismatches as much as possible when designing gRNA.

Normally, when introducing a CRISPR/Cas9 genome editing tool into a cell using a plasmid vector, a U6 promoter is normally used as the promoter expressing the gRNA. There are also limitations on the design of the target sequence since gRNA is not expressed at an adequate expression level unless the transcription starting point of this U6 promoter is a purine base (G or A).

On the other hand, although editing of a target gene sequence using HDR technology is normally carried out using single-stranded oligo-DNA (ssODN), double-stranded DNA (dsDNA) or a plasmid and the like as a HDR template donor, the efficiency thereof is extremely low (20% or less) and the design of the sequence of the homology arms of the template donor has also been clearly determined to be an important element in terms of carrying out genome editing with HDR.

The inventors of the present invention conducted extensive research to solve the above-mentioned problems. First, the inventors of the present invention succeeded in designing a special gRNA targeting a mutant TGFBI gene, which was not possible with ordinary design techniques, using a CRISPR/Cas9 system for the genome editing technology and then developing a ssODN having a total length of 100 nt capable of preventing effects attributable to re-cleavage as a HDR template donor as well as realizing easy confirmation of normalization of the TGFBI gene and gene repair. As a result, the inventors of the present invention discovered a means for repairing a mutant gene with high efficiency, high specificity and favorable reproducibility with respect to a mutation of the amino acid located at position 124 of the TGFBI protein of cells derived from corneal degeneration patients to restore to the normal TGFBI amino acid sequence, thereby leading to completion of the present invention.

Namely, the present application includes the inventions indicated below.
(1) A method of treating granular corneal degeneration in a subject, which comprises the step of:
   bringing keratocytes of the subject into contact with Cas9 and guide RNA in order to repair a mutation of a transforming growth factor β-induced (TGFBI) gene associated with granular corneal degeneration.
(2) The method described in (1), wherein the mutation of the TGFBI gene is a point mutation.
(3) The method described in (1) or (2), wherein the granular corneal degeneration is granular corneal dystrophy type 2 (GCD2).
(4) The method described in (2), wherein the point mutation is an amino acid substitution at position 124 of a TGFBI protein.
(5) The method described in (4), wherein the amino acid substitution is selected from the group consisting of R124H, R124C and R124L.
(6) The method described in any of (1) to (5), wherein the guide RNA comprises a guide sequence able to hybridize with a target sequence consisting of a 22-nucleotide sequence adjacent to an upstream side of a protospacer adjacent motif (PAM) sequence of the TGFBI gene.
(7) The method described in (6), wherein the PAM sequence consists of CGG.
(8) The method described in (6) or (7), wherein the guide sequence contains a region that consists of at least 17 to 18 nucleotides and is complementary to the target sequence.
(9) The method described in any of (6) to (8), wherein the guide sequence consists of the 22-nucleotide sequence starting from adenine or guanine.
(10) The method described in (9), wherein the guide sequence has a nucleotide sequence of SEQ ID NO: 1.
(11) The method described in any of (6) to (10), wherein the guide sequence is bound to a trans-activating crRNA (tracrRNA) sequence.
(12) The method described in any of (1) to (11), wherein expression of the guide RNA is driven by a U6 promoter.
(13) The method described in (12), wherein the guide RNA and the U6 promoter are located on an identical vector or different vectors.
(14) The method described in (13), wherein the guide RNA and the U6 promoter are operably linked on an identical vector.
(15) The method described in any of (1) to (14), wherein correction of the mutation of the TGFBI gene is carried out through homology directed repair (HDR) of a sequence cleaved by Cas9.
(16) The method described in (15), wherein single-stranded oligo-DNA (ssODN) is used as an HDR template donor.
(17) The method described in (16), wherein the ssODN comprises a knock-in sequence in which a codon including a point mutation is replaced with a codon including nucleotides corresponding to a wild-type amino acid.
(18) The method described in (17), wherein the nucleotides corresponding to the wild-type amino acid are CGT or CGC.
(19) The method described in (18), wherein the knock-in sequence comprises a restriction enzyme site.
(20) The method described in (19), wherein the nucleotides corresponding to the wild-type amino acid are CGT and the restriction enzyme site is a BsiWI site.
(21) The method described in any of (17) to (20), wherein the ssODN further comprises, on both ends of the knock-in sequence, 50-nucleotide homology arms each homologous to either end of a cleavage site.
(22) The method described in any of (1) to (21), wherein the Cas9 and the gRNAform a ribonucleoprotein (RNP) complex when being in contact with the cells.
(23) A guide RNA molecule that hybridizes with a target sequence containing a mutation site of a TGFBI gene associated with granular corneal degeneration.
(24) The guide RNA molecule described in (23), wherein the mutation of the TGFBI gene is a point mutation.
(25) The guide RNA molecule described in (23) or (24), wherein the granular corneal degeneration is GCD2.
(26) The guide RNA molecule described in any of (23) to (25), wherein the point mutation is an amino acid substitution at position 124 of a TGFBI protein.
(27) The guide RNA molecule described in (26), wherein the amino acid substitution is selected from the group consisting of R124H, R124C and R124L.
(28) The guide RNA molecule described in any of (23) to (28), including a guide sequence able to hybridize with a target sequence consisting of a 22-nucleotide sequence adjacent to an upstream side of a PAM sequence of the TGFBI gene.
(29) The guide RNA molecule described in (28), wherein the PAM sequence consists of CGG.
(30) The guide RNA molecule described in (28) or (29), wherein the guide sequence contains a region that consists of at least 17 to 18 nucleotides and is complementary to the target sequence.
(31) The guide RNA molecule described in any of (28) to (30), wherein the guide sequence consists of the 22-nucleotide sequence starting from adenine or guanine.
(32) The guide RNA molecule described in any of (28) to (31), wherein the guide sequence has a nucleotide sequence of SEQ ID NO: 1.
(33) The guide RNA molecule described in any of (28) to (32), wherein the guide sequence is bound to a tracrRNA sequence.
(34) A nucleic acid encoding the guide RNA molecule described in any of (23) to (33).
(35) A vector that expresses the guide RNA molecule described in any of (23) to (33).
(36) The vector described in (35), wherein expression of the guide RNA is driven by a U6 promoter.
(37) The vector described in (36), wherein the guide RNA and the U6 promoter are located on an identical vector or different vectors.
(38) The vector described in (36), wherein the guide RNA and the U6 promoter are operably linked on an identical vector.
(39) A kit, including:
   the vector described in any of (35) to (38); and
   a ssODN molecule as an HDR template donor.
(40) The kit described in (39), wherein the ssODN comprises a knock-in sequence in which a codon including a point mutation is replaced with a codon including nucleotides corresponding to a wild-type amino acid.
(41) The kit described in (40), wherein the nucleotides corresponding to the wild-type amino acid are CGT or CGC.
(42) The kit described in (40) or (41), wherein the knock-in sequence comprises a restriction enzyme site.
(43) The kit described in (42), wherein the nucleotides corresponding to the wild-type amino acid are CGT and the restriction enzyme site is a BsiWI site.
(44) The kit described in any of (39) to (43), wherein the ssODN further comprises, on both ends of the knock-in sequence, 50-nucleotide homology arms each homologous to either end of a cleavage site.
(45) The kit described in any of (39) to (44), wherein the ssODN molecule has a nucleotide sequence of SEQ ID NO: 2.
(46) A kit, including:
   the guide RNA molecule described in any of (23) to (33); and
   a Cas9 protein.
(47) The kit described in (46), wherein the guide RNA molecule and the Cas9 protein form a ribonucleoprotein (RNP) complex.
(48) A pharmaceutical composition, including any one or more of the following:
   the guide RNA molecule described in any of (23) to (33);
   the nucleic acid encoding the guide RNA molecule described in (34); or
   a ssODN molecule as an HDR template donor, the ssODN molecule having the nucleotide sequence of SEQ ID NO: 2.

### Advantageous Effects of Invention

In particular, the present inventors developed a 22 nt gRNA having a mutation sequence-specific nucleic acid sequence by using, as a PAM sequence, a CGG sequence located 4 bp downstream from a cleavage site in order to cleave a gene sequence corresponding to the 124th amino acid of a mutated TGFBI gene. In addition, although gRNA used for SpCas9 is normally designed to have a length of 20 nt, in the case of cleaving the gene sequence corresponding to the 124th amino acid, the sequence at the transcription starting point of the gRNA becomes T due to restrictions on the PAM sequence, thereby resulting in the problem of causing decreases in the expression level of gRNA and cleavage efficiency. Moreover, since there are numerous off-target sequences of 3 nt mismatches with respect to this conventional gRNA designed at a length of 20 nt, there is also the possibility of problems resulting from non-specific sequence cleavage. On the basis thereof, the cleavage of such a gene sequence with high efficiency and high specificity using a CRISPR/Cas9 system was not possible with the technology of the prior art.

### Brief Description of Drawings

Fig. 1 shows a comparison of an example of a mutation of the TGFBI gene in corneal degeneration with the wild type (Wild type/Wild type: wild-type amino acid sequence represented by SEQ ID NO: 3, nucleotide sequence represented by SEQ ID NO: 4; Wild type/R124H: amino acid sequence of heterozygous mutation represented by SEQ ID NO: 5, nucleotide sequence represented by SEQ ID NO: 6), in which the gene corresponding to the 124th amino acid of the TGFBI gene has mutated.
Fig. 2 shows each of the sequences of hTGFBI R124H gRNA and hTGFBI R124H-repaired ssODN introduced with a BsiWI site (C/GTACG) along with their surrounding sequences.
Fig. 3 shows a diagram schematically showing the structure of a mutant TGFBI gene-specific CRISPR/Cas9 targeting plasmid vector.
Fig. 4 shows results confirming knock-in by PCR-RFLP using BsiWI.
Fig. 5 shows the results of genetic analysis of post-knock-in sequences.
Fig. 6 depicts results indicating that mono-allelic editing was observed in 13 of 63 clones (20.6%) by knock-in and that bi-allelic editing was confirmed in 26 clones (41.3%).
Fig. 7a shows the results of analyzing genomic sequences serving as off-target candidates; Fig. 7b shows genomic sequences serving as off-target candidates; and Fig. 7c shows the results of amplifying genomic sequences for the off-target sequences by PCR and analyzing using a T7 endonuclease I assay.

### Description of Embodiments

### (Treatment Method for Granular Corneal Degeneration)

In one aspect thereof, the present invention provides a method of treating granular corneal degeneration by repairing a mutation of the TGFBI gene through a genome editing mechanism. In the present invention, it is intended to repair any mutation of the TGFBI gene, such as a point mutation, that is involved in granular corneal degeneration.

In granular corneal dystrophy type 2 (GCD2), which is a type of granular corneal degeneration, arginine located at position 124 of the TGFBI protein is replaced with another amino acid such as histidine. Other point mutations known to cause GCD2 in addition to R124H include R124C and R124L.

Theoretically, granular corneal degeneration can be treated by replacing an abnormality in the nucleotide sequence corresponding to a point mutation in the TGFBI protein with the normal nucleotide sequence. However, in order to realize genome editing technology that has high efficiency, high specificity and favorable reproducibility and, in turn, reliably treat granular corneal degeneration, it is necessary to introduce a genome editing tool into cells with high efficiency and precisely design the genome editing tool.

Among genome editing tools, an explanation of RNA-guided CRISPR/Cas9 in particular is provided by using as an example the case of using the commonly used RNA-guided nuclease, Cas9 (SpCas9), derived from Streptococcus pyogenes. In this RNA-guided CRISPR/Cas9, any DNA sequence complementarily bound to gRNA is subjected to double-strand cleavage using a complex of SpCas9 protein and RNA of about 120 nucleotides having a sequence required to recognize a 20 base target sequence referred to as guide RNA (gRNA).

In the present invention, in order to cleave a target sequence in the TGFBI gene involved in granular corneal degeneration, keratocytes obtained from a subject afflicted with granular corneal degeneration are contacted with Cas protein and gRNA. Although the Cas protein and gRNA may be added separately, they are preferably used in the form of a ribonucleoprotein (RNP) complex in order to suppress off-target effects.

Although there are no limitations on the Cas protein used in the present invention provided it belongs to a CRISPR system, Cas9 is preferable. Examples of Cas9 include Cas9 derived from Streptococcus pyogenes (SpCas9) and Cas9 derived from Streptococcus thermophilus (StCas9). Among these, since there are limitations on the PAM sequence when cleaving a target site, SpCas9 that is commonly used for genome editing is preferable. The Cas protein and gRNA are delivered into cells via a vector encoding them using a method known among persons with ordinary skill in the art such as standard transfection methods, electroporation or lentivirus transduction. The vector for expressing Cas and the vector for expressing gRNA may be the same vector or mutually different vectors. In addition, a complex of recombinant Cas9 protein and gRNA (RNP, ribonucleoprotein) can be formed and the RNP can be delivered directly into the cells by a method known among persons with ordinary skill in the art such as transduction or electroporation.

Keratocytes are flat cells present in the corneal stromal layer that are important for maintaining transparency of the cornea. Each cell has projections and demonstrates a network structure that contacts adjacent keratocytes by means of those projections. In keratocytes of granular corneal degeneration, transparency is impaired due to accumulation of mutant TGFBI protein. Keratocytes are obtained during surgical procedures such as deep anterior lamellar keratoplasty by removing the corneal epithelium followed by harvesting keratocytes from the interstitium and treating with collagenase.

Gene transfer into the keratocytes of a subject uses a method known among persons with ordinary skill in the art such as targeted transduction, electroporation or viral vector such as that of adeno-associated virus. The cornea is a tissue that enables DNA to be introduced into the cells by naked DNA delivery without using a special transfection reagent, and can be delivered directly into cells by direct injection of DNA (Brain Res. Bull. 2010 Feb. 15; 81(2-3): 256-261). In addition, after having formed the complex of recombinant Cas9 protein and gRNA, the complex can be delivered into cells using a method known among persons with ordinary skill in the art such as transduction or electroporation.

The subject is not limited to a mammal such as a human, monkey, rhesus monkey, marmoset, orangutan or chimpanzee, and is intended to be, for example, a laboratory animal such as a mouse, rat, hamster, guinea pig or other rodent or a rabbit, an even-toed ungulate such as a pig, cow, goat or sheep, an odd-toed ungulate such as a horse, or a pet such as a dog or cat. However, the subject is preferably a human corneal degeneration patient.

Cas9, which is a DNA cleaving enzyme, functions as a DNA endonuclease that is able to cleave DNA at a site where target DNA is present. gRNA has a sequence complementary to a target DNA on the 5'-end thereof and has the function of guiding Cas9 to the target DNA by binding to the target DNA through that complementary sequence. Furthermore, Cas9 protein and gRNA form a complex in about 10 minutes under room temperature conditions that immediately demonstrates the ability to cleave target DNA.

The design of the gRNA is important in order to carry out double-strand cleavage of a target sequence with high efficiency. For example, three nucleotides consisting of NGG referred to as a protospacer adjacent motif (PAM) sequence are required to be present immediately after a 20 base target sequence. In addition, since double-strand cleavage by SpCas9 occurs 3 to 4 nucleotides upstream from this PAM sequence, in the case the site desired to be cleaved has been determined, double-strand cleavage at the site using SpCas9 is not possible unless the PAM sequence is downstream therefrom. In the case the site to be cleaved is a gene sequence corresponding to the 124th amino acid of the TGFBI gene, a CGG sequence present four nucleotides downstream from that gene sequence can be used as a PAM sequence.

When designing gRNA, a person with ordinary skill in the art would be able to suitably select the GC content in the nucleotide sequence to be within the range of 40% to 80%. A higher GC content, such as a GC content of 50% or more, is preferable from the viewpoint of improving cleavage efficiency.

gRNA is further designed so as to reduce as much as possible non-specific cleavage caused by off-target effects. For example, although nucleotide length may be the normally used 20 nucleotides, numerous off-target candidates may be found. In this case, increasing the nucleotide length to 22 nucleotides considerably reduces the number of off-target sequences thereby making it possible to provide gRNA having high specificity in that off-target sequences extending to three base mismatches are not present in the human genome.

In order to express an adequate level of gRNA, it is necessary to select a suitable promoter to drive that expression. Although RNA polymerase III-dependent U6 promoter or T7 promoter, for example, are normally used for this promoter, it is necessary to design the gRNA according to the promoter used. For example, in the case of using the U6 promoter, since gRNA is not expressed at an adequate expression level unless the transcription starting point is a purine base (G or A), in the case of expressing gRNA in the presence of U6 promoter, the gRNA preferably consists of a nucleotide sequence starting from adenine or guanine.

gRNA can normally be introduced into cells in a state of being arranged in one or more plasmid vectors together with a suitable promoter. In this case, the guide RNA and promoter may be operably linked on the same vector or may be present on different vectors.

The guide sequence is designed so as to contain a region consisting of at least 17 to 18 nucleotides complementary to a target sequence so as to be able to hybridize with the target sequence. In the present description, a target sequence refers to a region containing a mutation site of the TGFBI gene, such as a sequence of 22 nucleotides adjacent to the upstream side of a PAM sequence.

In a more preferable aspect, the gRNA is designed to have the nucleotide sequence of SEQ ID NO: 1: 5'-acucagcuguacacggaccaca-3'. This gRNA can be chemically synthesized.

The gRNA is further bound to a trans-activating crRNA (tracrRNA) sequence in order to recruit Cas protein.

After having cleaved the both strands of a target sequence containing a mutation site, the mutation site can be repaired by knocking in donor DNA containing the normal sequence at the cleavage site. The donor DNA can be introduced into cells simultaneous to Cas9 mRNA and gRNA. Homology directed repair (HDR) is possible since the donor is present during the formation of a double strand break (DSB).

Although there are various methods for carrying out knock-in, knock-in mediated by homologous recombination (HR) is preferable due to the high correction accuracy thereof. Genetic information on a chromosome containing a mutation site can be substituted for novel information derived from a donor by carrying out homologous recombination in the presence of donor DNA encoding the normal sequence. Although the donor DNA may be any of ssODN, dsDNA or a plasmid, in the case of substituting a single nucleotide polymorphism in the manner of R124H, it is preferable to repair the mutation site using a ssODN containing the normal sequence.

Donor DNA for repairing a point mutation has: 1) a knock-in sequence in which the point mutation is replaced with a base corresponding to the wild-type amino acid, and 2) homology arms consisting of a "left arm" and a "right arm" identical to the sequence adjacent to both ends of the sequence to be replaced. Although the nucleotide length of the homology arms is preferably about 50 nucleotides, nucleotide lengths of about 40 nucleotides or about 60 nucleotides are not intended to be excluded, and a person with ordinary skill in the art would be able to suitably change the length of the homology arms.

The donor DNA optionally has a restriction enzyme site. Confirmation of genetic modification efficiency can be simplified by providing a restriction enzyme site in the donor DNA. In providing an explanation of this using as an example the case of repairing a point mutation located at position 124 of the TGFBI protein, although the nucleotides corresponding to the wild-type amino acid are CGC, by containing, for example, CGT instead of CGC in the knock-in sequence, a BsiWI site (C/GTACG) can be introduced as a restriction enzyme site.

Required sequences other than a restriction enzyme site, such as a reporter gene or drug resistance gene, may also be introduced into the donor DNA. For example, the normal sequence is easily detected if a reporter gene is linked in-frame to the C-end of the normal sequence. Examples of reporter genes include genes encoding fluorescent proteins such as green fluorescent protein (GFP), humanized Renilla reniformis green fluorescent protein (hrGFP) or enhanced green fluorescent protein (eGFP), and genes encoding bioluminescent proteins such as firefly luciferase or Renilla reniformis luciferase.

The ssODN represented by SEQ ID NO: 2 has a BsiWI site for the restriction enzyme site and homology arms consisting of 50 nucleotides for each arm, and is able to prevent effects attributable to re-cleavage as a homologous recombination repair HDR template donor while also making it possible to easily confirm normalization of the TGFBI gene and gene repair.

### (Guide RNA and Application Thereof)

In one aspect thereof, the present invention further provides a guide RNA molecule that hybridizes with a target sequence containing a mutation site of the TGFBI gene and/or a nucleic acid that encodes the same, or an application thereof. Details such as the guide sequence of the guide RNA molecule are as previously described.

In another aspect, the present invention provides a vector containing a nucleic acid and the like that encodes a guide RNA molecule. The vector provided by the present invention may be a circular vector or a linear vector. The vector provided by the present invention is preferably a circular vector. Examples of the vector of the present invention include plasmid vectors, cosmid vectors, viral vectors and artificial chromosome vectors. Examples of artificial chromosome vectors include a yeast artificial chromosome (YAC) vector, bacterial artificial chromosome (BAC) vector, P1-derived artificial chromosome (PAC) vector, mouse artificial chromosome (MAC) vector and human artificial chromosome (HAC) vector.

The vector encoding the guide RNA and the vector for expressing Cas endonuclease may be the same vector or mutually different vectors. In addition, these vectors and the vector encoding donor DNA such as a ssODN may also be the same or different.

In one aspect thereof, the present invention provides a kit containing one or a plurality of vectors encoding the above-mentioned guide RNA molecule or nucleic acid encoding the same and/or a template donor (such as a ssODN) or nucleic acid encoding the same. The kit of the present invention can be preferably used to treat granular corneal degeneration. In addition, the kit of the present invention can also be used in a diagnostic technology by detecting a repaired TGFBI gene by editing the genome for a mutant TGFBI gene followed by amplifying the region containing that sequence by PCR and the like and digesting with BsiWI using a BsiWI restriction enzyme cleavage sequence inserted into the gene by HDR.

In another aspect, the present invention provides a pharmaceutical composition containing the above-mentioned guide RNA molecule or nucleic acid encoding the same and/or a template donor molecule (such as a ssODN) or nucleic acid encoding the same. The pharmaceutical composition of the present invention can be preferably used to treat granular corneal degeneration.

Although the following provides a more detailed explanation of the present invention by listing examples and comparative examples, the present invention is not limited thereto.

### Examples

The gRNA of the subject patent invention was designed in consideration of precautions to be taken when designing gRNA as previously described (SEQ ID NO: 1). For example, since numerous off-target sequences are found if the nucleotide length is the normally used 20 nucleotides, the number off-target sequences was significantly reduced by increasing nucleotide length to 22 nucleotides. The GC content of the gRNA of SEQ ID NO: 1 is 50% or more, which is the ideal value in terms of forming a complex with spCas9 and cleaving the target sequence.

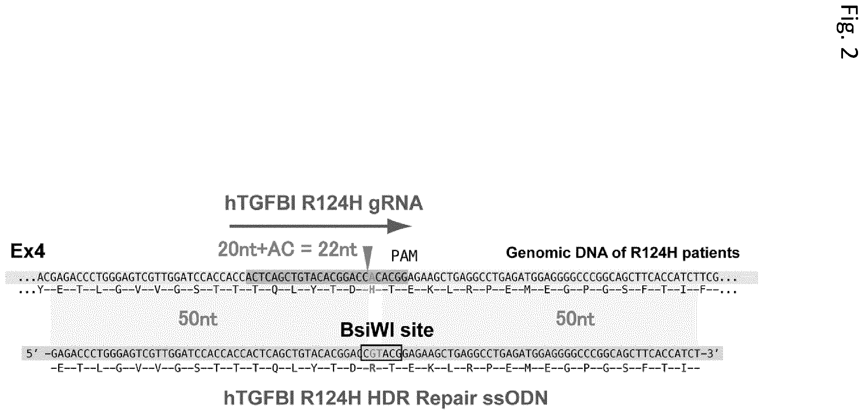

In addition, a ssODN having 50 bp homology arms homologous to the cleavage region on both ends (5'-GAGACCCTGGGAGTCGTTGGATCCACCACCACTCAGCTGTACACGGACCGTAC GGAGAAGCTGAGGCCTGAGATGGAGGGGCCCGGCAGCTTCACCATCT-3' (SEQ ID NO: 2)) was designed for use as an HDR template donor. In addition, the HDR template donor was designed to enable the introduction of a codon (CGT) in which the nucleotide sequence introduced into the TGFBI gene mutation site differs from arginine (CGC) of wild-type TGFBI, thereby enabling normalization of the gene sequence, simplification of confirmation of gene modification efficiency (by introducing a BsiWI restriction enzyme site), and preventing re-cleavage following gene sequence normalization (Fig. 2).

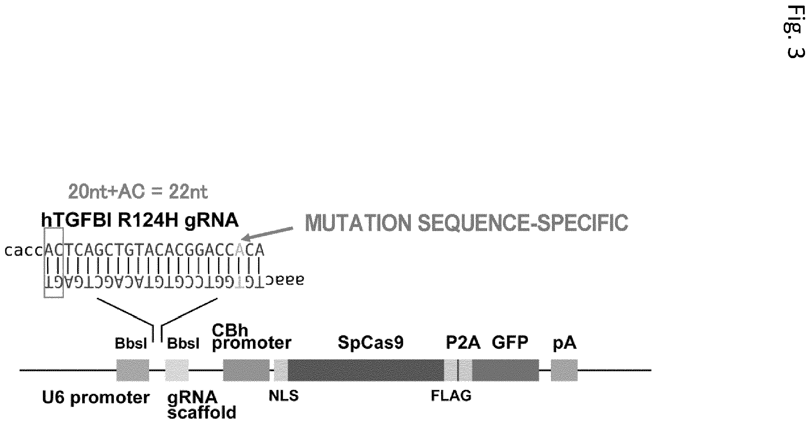

The mutant TGFBI gene-specific gRNA sequence (SEQ ID NO: 1) was introduced into a pX458 vector (Addgene: Cat. No. 48138) enabling simultaneous expression of gRNA and SpCas9-2A-GFP gene to produce a mutant TGFBI gene-specific CRISPR/Cas9 targeting plasmid vector (Fig. 3). The DNA nucleotide sequence of the plasmid is represented by SEQ ID NO: 7 and the RNA nucleotide sequence of the guide RNA indicated below is represented by SEQ ID NO. pX458-hTGFBI(R124H) gRNA, RNA nucleotide sequence (total length): 5-ACUCAGCUGUACACGGACCACAguuuuagagcuaGAAAuagcaaguuaaaauaaggcuag uccguuaucaacuugaaaaaguggcaccgagucggugcuuuu-3'

Continuing, the above-mentioned plasmid vector was transferred to keratocytes derived from human corneal degeneration patients together with a ssODN and HDR template donor (SEQ ID NO: 2) followed one week later by separating the transferred cells based on expression of GFP gene. After culturing and amplifying the resulting cells, genome editing efficiency was confirmed by carrying out restriction fragment length polymorphism (RFLP) analysis and gene sequence analysis.

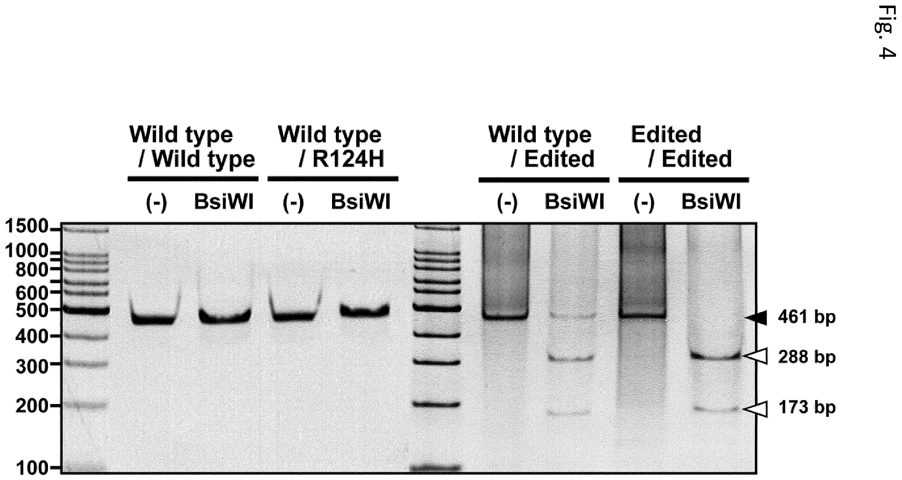

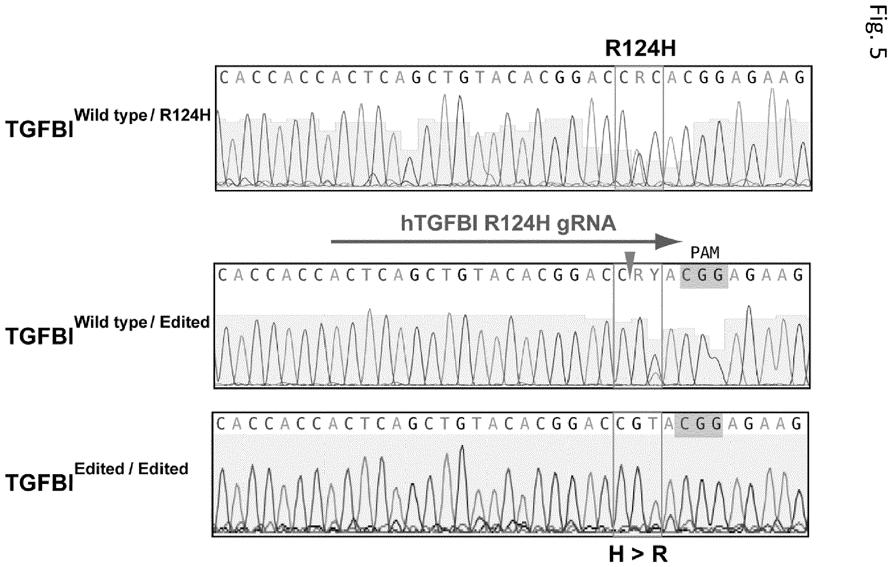

As a result, a mutation of the TGFBI gene was successfully restored to the normal sequence in keratocytes derived from human corneal degeneration patients by introducing a mutant TGFBI gene-specific CRISPR/Cas9 targeting plasmid vector and HDR template ssODN (Figs. 4 and 5).

Genome editing efficiency was confirmed by PCR-amplifying the gene sequence corresponding to the R124H mutation site of the TGFBI gene and carrying out RFLP and gene sequence analyses. The PCR primer sequences used for gene sequence analysis of the TGFBI mutation site by PCR are indicated below. Forward: 5'-GTTGAGTTCACGTAGACAGGC-3' (SEQ ID NO: 9) Reverse: 5'-GACTCCCATTCATCATGCCCA-3' (SEQ ID NO: 10)

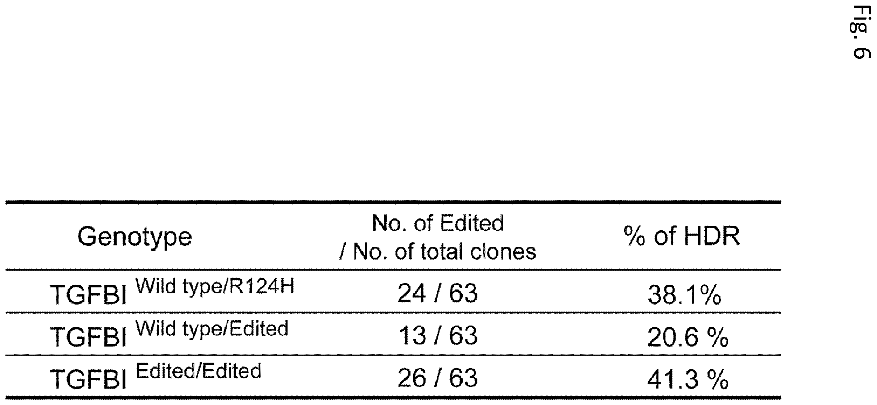

As a result, in contrast to a 461 bp band having been detected in the TGFBI wild-type keratocytes serving as a control, in the keratocytes derived from human corneal degeneration patients that underwent genome editing, a BsiWI restriction enzyme site was introduced by genome editing and BsiWI digestion resulted in cleaved 288 bp and 173 bp bands. When this analysis was carried out on all 89 clones that underwent gene transfer, mono-allelic editing was confirmed in 13 of 63 clones (20.6%) and bi-allelic editing was confirmed in 26 clones (41.3%). On the basis of these results, use of the present invention was determined to enable a mutation of the TGFBI gene in keratocytes derived from human corneal degeneration patients to be restored to normal at the extremely high genome editing efficiency of a total of 61.9% (Fig. 6).

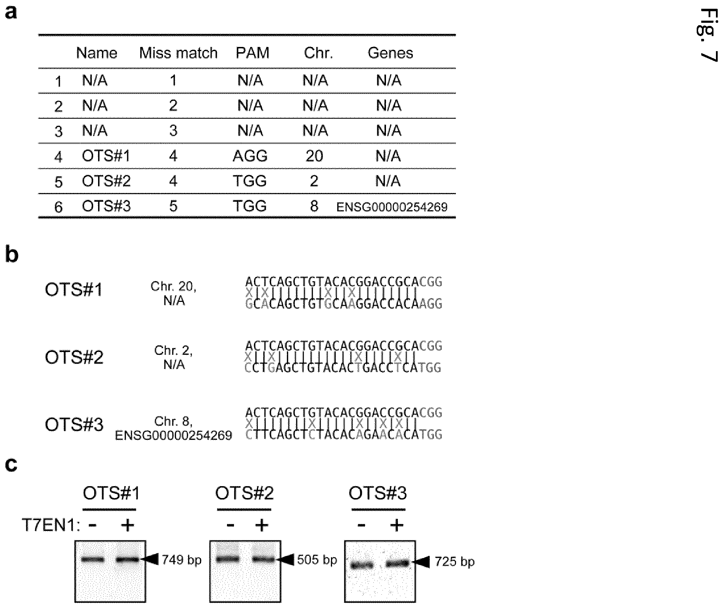

Moreover, according to the present invention, the number of off-target candidates was able to be reduced to a greater extent than gRNA designed according to the prior art. Although gRNA has been reported to typically not demonstrate cleavage activity with respect to sequences differing by three nucleotides from the target sequence thereof (Nature Biotechnology 31, 822-826 (2013)), as a result of analyzing off-target sequences with respect to the designed gRNA, it was found that no sequences containing mismatches of 1 to 3 nucleotides with respect to the designed gRNA were present (Fig. 7a). On the other hand, sequences in which 4 or 5 nucleotides were mismatched were confirmed in three chromosome regions (chromosome #20 24871179-24871203, chromosome #2 98321072-98321097 and chromosome #8 1150445-1150469) (Figs. 7a and 7b). The genome sequences containing these sequences were amplified by PCR and analyzed by an ordinary T7 endonuclease I assay. The sets of PCR primers used in off-target analysis are indicated below.
OTS1
   5'-ATGTCAGAAGTCCCGCTGTG-3' (SEQ ID NO: 11)
   5'-TGATGGGGTCAGAGGGCATA-3' (SEQ ID NO: 12)
OTS2
   5'-CTTCCTGCTCTGTGTTTAGCCA-3' (SEQ ID NO: 13)
   5'-ACCTCCAAGTTGAGCAGTGTC-3' (SEQ ID NO: 14)
OTS3
   5'-GCAGCAAAGCACTCAAGAGG-3' (SEQ ID NO: 15)
   5'-CAAACTTCTGCCTGGGCATC-3' (SEQ ID NO: 16)

T7 endonuclease I recognizes double-stranded mismatches between PCR amplification products derived from off-target cleavage and wild-type products and cleaves heteroduplex mismatched portions. As a result, cleavage by T7 endonuclease I was not detected in any of the PCR products. In other words, non-specific cleavage caused by off-target effects was not observed for any of the sequences (Fig. 7c).

Based on the above results, these findings suggested that the mutant TGFBI gene-specific gRNA according to the present invention is able to actually reduce cleavage caused by off-target effects.

## Claims

1. A guide RNA molecule that hybridizes with a target sequence containing a mutation site of a transforming growth factor β-induced (TGFBI) gene associated with granular corneal degeneration.

2. The guide RNA molecule according to claim 1, wherein the mutation of the TGFBI gene is a point mutation.

3. The guide RNA molecule according to claim 2, wherein the point mutation is an amino acid substitution at position 124 of a TGFBI protein.

4. The guide RNA molecule according to claim 3, wherein the amino acid substitution is selected from the group consisting of R124H, R124C and R124L.

5. The guide RNA molecule according to any of claims 1 to 4, wherein the granular corneal degeneration is granular corneal dystrophy type 2 (GCD2).

6. The guide RNA molecule according to any of claims 1 to 5, comprising a guide sequence able to hybridize with a target sequence consisting of a 22-nucleotide sequence adjacent to an upstream side of a protospacer adjacent motif (PAM) sequence of the TGFBI gene.

7. The guide RNA molecule according to claim 6, wherein the PAM sequence consists of CGG.

8. The guide RNA molecule according to claim 6 or 7, wherein the guide sequence contains a region that consists of at least 17 to 18 nucleotides and is complementary to the target sequence.

9. The guide RNA molecule according to any of claims 6 to 8, wherein the guide sequence consists of the 22-nucleotide sequence starting from adenine or guanine.

10. The guide RNA molecule according to any of claims 6 to 9, wherein the guide sequence has a nucleotide sequence of SEQ ID NO: 1.

11. The guide RNA molecule according to any of claims 6 to 10, wherein the guide sequence is bound to a trans-activating crRNA (tracrRNA) sequence.

12. A nucleic acid encoding the guide RNA molecule according to any of claims 1 to 11.

13. A vector that expresses the guide RNA molecule according to any of claims 1 to 11.

14. The vector according to claim 13, wherein expression of the guide RNA is driven by a U6 promoter.

15. The vector according to claim 14, wherein the guide RNA and the U6 promoter are located on an identical vector or different vectors.

16. The vector according to claim 14, wherein the guide RNA and the U6 promoter are operably linked on an identical vector.

17. A kit, comprising:
the vector according to any of claims 13 to 16; and
a ssODN molecule as an HDR template donor.

18. The kit according to claim 17, wherein the ssODN comprises a knock-in sequence in which a codon including a point mutation is replaced with a codon including nucleotides corresponding to a wild-type amino acid.

19. The kit according to claim 18, wherein the nucleotide corresponding to the wild-type amino acid are CGT or CGC.

20. The kit according to claim 18 or 19, wherein the knock-in sequence comprises a restriction enzyme site.

21. The kit according to claim 20, wherein the nucleotides corresponding to the wild-type amino acid are CGT and the restriction enzyme site is a BsiWI site.

22. The kit according to any of claims 17 to 21, wherein the ssODN further comprises, on both ends of the knock-in sequence, 50-nucleotide homology arms each homologous to either end of a cleavage site.

23. The kit according to any of claims 17 to 22, wherein the ssODN molecule has a nucleotide sequence of SEQ ID NO: 2.

24. A kit, comprising:
the guide RNA molecule according to any of claims 1 to 11; and
a Cas9 protein.

25. The kit according to claim 24, wherein the guide RNA molecule and the Cas9 protein form a ribonucleoprotein (RNP) complex.

26. A pharmaceutical composition, comprising:
the guide RNA molecule according to any of claims 1 to 11 or the nucleic acid according to claim 12; and/or
a ssODN molecule as an HDR template donor, the ssODN molecule having a nucleotide sequence of SEQ ID NO: 2.


SEQUENCE LISTING

<110>  The University of Tokyo
       Chiba University

<120>  Gene Therapeutic Agent for Granular Corneal Dystrophy

<130>  T0529AJP0006

<150>  JP 2017-112406
<151>  2017-06-07

<160>  16

<170>  PatentIn version 3.5

<210>  1
<211>  22
<212>  RNA
<213>  Artificial Sequence

<220>
<223>  gRNA

<400>  1
acucagcugu acacggacca ca                                              22


<210>  2
<211>  100
<212>  DNA
<213>  Artificial Sequence

<220>
<223>  ssODN

<400>  2
gagaccctgg gagtcgttgg atccaccacc actcagctgt acacggaccg tacggagaag      60

ctgaggcctg agatggaggg gcccggcagc ttcaccatct                          100


<210>  3
<211>  683
<212>  PRT
<213>  Homo sapiens

<400>  3

<210> 4
<211> 2052
<212> DNA
<213> Homo sapiens

<400> 4
atggcgctct tcgtgcggct gctggctctc gccctggctc tggccctggg ccccgccgcg 60

accctggcgg gtcccgccaa gtcgccctac cagctggtgc tgcagcacag caggctccgg 120

ggccgccagc acggccccaa cgtgtgtgct gtgcagaagg ttattggcac taataggaag 180

tacttcacca actgcaagca gtggtaccaa aggaaaatct gtggcaaatc aacagtcatc 240

agctacgagt gctgtcctgg atatgaaaag gtccctgggg agaagggctg tccagcagcc 300

ctaccactct caaaccttta cgagaccctg ggagtcgttg gatccaccac cactcagctg 360

tacacggacc gcacggagaa gctgaggcct gagatggagg ggcccggcag cttcaccatc 420

ttcgccccta gcaacgaggc ctgggcctcc ttgccagctg aagtgctgga ctccctggtc 480

agcaatgtca acattgagct gctcaatgcc ctccgctacc atatggtggg caggcgagtc 540

ctgactgatg agctgaaaca cggcatgacc ctcacctcta tgtaccagaa ttccaacatc 600

cagatccacc actatcctaa tgggattgta actgtgaact gtgcccggct gctgaaagcc 660

gaccaccatg caaccaacgg ggtggtgcac ctcatcgata aggtcatctc caccatcacc 720

aacaacatcc agcagatcat tgagatcgag gacacctttg agacccttcg ggctgctgtg 780

gctgcatcag ggctcaacac gatgcttgaa ggtaacggcc agtacacgct tttggccccg 840

accaatgagg ccttcgagaa gatccctagt gagactttga accgtatcct gggcgaccca 900

gaagccctga gagacctgct gaacaaccac atcttgaagt cagctatgtg tgctgaagcc 960

atcgttgcgg ggctgtctgt agagaccctg gagggcacga cactggaggt gggctgcagc 1020

ggggacatgc tcactatcaa cgggaaggcg atcatctcca ataaagacat cctagccacc 1080

aacggggtga tccactacat tgatgagcta ctcatcccag actcagccaa gacactattt 1140

gaattggctg cagagtctga tgtgtccaca gccattgacc ttttcagaca agccggcctc 1200

ggcaatcatc tctctggaag tgagcggttg accctcctgg ctcccctgaa ttctgtattc 1260

aaagatggaa cccctccaat tgatgcccat acaaggaatt tgcttcggaa ccacataatt 1320

aaagaccagc tggcctctaa gtatctgtac catggacaga ccctggaaac tctgggcggc 1380

aaaaaactga gagtttttgt ttatcgtaat agcctctgca ttgagaacag ctgcatcgcg 1440

gcccacgaca agagggggag gtacgggacc ctgttcacga tggaccgggt gctgaccccc 1500

ccaatgggga ctgtcatgga tgtcctgaag ggagacaatc gctttagcat gctggtagct 1560

gccatccagt ctgcaggact gacggagacc ctcaaccggg aaggagtcta cacagtcttt 1620

gctcccacaa atgaagcctt ccgagccctg ccaccaagag aacggagcag actcttggga 1680

gatgccaagg aacttgccaa catcctgaaa taccacattg gtgatgaaat cctggttagc 1740

ggaggcatcg gggccctggt gcggctaaag tctctccaag gtgacaagct ggaagtcagc 1800

ttgaaaaaca atgtggtgag tgtcaacaag gagcctgttg ccgagcctga catcatggcc 1860

acaaatggcg tggtccatgt catcaccaat gttctgcagc ctccagccaa cagacctcag 1920

gaaagagggg atgaacttgc agactctgcg cttgagatct tcaaacaagc atcagcgttt 1980

tccagggctt cccagaggtc tgtgcgacta gcccctgtct atcaaaagtt attagagagg 2040

atgaagcatt ag 2052

<210> 5
<211> 683
<212> PRT
<213> Homo sapiens

<400> 5

<210>  6
<211>  2052
<212>  DNA
<213>  Homo sapiens

<400>  6
atggcgctct tcgtgcggct gctggctctc gccctggctc tggccctggg ccccgccgcg      60

accctggcgg gtcccgccaa gtcgccctac cagctggtgc tgcagcacag caggctccgg     120

ggccgccagc acggccccaa cgtgtgtgct gtgcagaagg ttattggcac taataggaag     180

tacttcacca actgcaagca gtggtaccaa aggaaaatct gtggcaaatc aacagtcatc     240

agctacgagt gctgtcctgg atatgaaaag gtccctgggg agaagggctg tccagcagcc     300

ctaccactct caaaccttta cgagaccctg ggagtcgttg gatccaccac cactcagctg     360

tacacggacc acacggagaa gctgaggcct gagatggagg ggcccggcag cttcaccatc     420

ttcgccccta gcaacgaggc ctgggcctcc ttgccagctg aagtgctgga ctccctggtc     480

agcaatgtca acattgagct gctcaatgcc ctccgctacc atatggtggg caggcgagtc     540

ctgactgatg agctgaaaca cggcatgacc ctcacctcta tgtaccagaa ttccaacatc     600

cagatccacc actatcctaa tgggattgta actgtgaact gtgcccggct gctgaaagcc     660

gaccaccatg caaccaacgg ggtggtgcac ctcatcgata aggtcatctc caccatcacc 720

aacaacatcc agcagatcat tgagatcgag gacacctttg agacccttcg ggctgctgtg 780

gctgcatcag ggctcaacac gatgcttgaa ggtaacggcc agtacacgct tttggccccg 840

accaatgagg ccttcgagaa gatccctagt gagactttga accgtatcct gggcgaccca 900

gaagccctga gagacctgct gaacaaccac atcttgaagt cagctatgtg tgctgaagcc 960

atcgttgcgg ggctgtctgt agagaccctg gagggcacga cactggaggt gggctgcagc 1020

ggggacatgc tcactatcaa cgggaaggcg atcatctcca ataaagacat cctagccacc 1080

aacggggtga tccactacat tgatgagcta ctcatcccag actcagccaa gacactattt 1140

gaattggctg cagagtctga tgtgtccaca gccattgacc ttttcagaca agccggcctc 1200

ggcaatcatc tctctggaag tgagcggttg accctcctgg ctcccctgaa ttctgtattc 1260

aaagatggaa cccctccaat tgatgcccat acaaggaatt tgcttcggaa ccacataatt 1320

aaagaccagc tggcctctaa gtatctgtac catggacaga ccctggaaac tctgggcggc 1380

aaaaaactga gagtttttgt ttatcgtaat agcctctgca ttgagaacag ctgcatcgcg 1440

gcccacgaca agagggggag gtacgggacc ctgttcacga tggaccgggt gctgaccccc 1500

ccaatgggga ctgtcatgga tgtcctgaag ggagacaatc gctttagcat gctggtagct 1560

gccatccagt ctgcaggact gacggagacc ctcaaccggg aaggagtcta cacagtcttt 1620

gctcccacaa atgaagcctt ccgagccctg ccaccaagag aacggagcag actcttggga 1680

gatgccaagg aacttgccaa catcctgaaa taccacattg gtgatgaaat cctggttagc 1740

ggaggcatcg gggccctggt gcggctaaag tctctccaag gtgacaagct ggaagtcagc 1800

ttgaaaaaca atgtggtgag tgtcaacaag gagcctgttg ccgagcctga catcatggcc 1860

acaaatggcg tggtccatgt catcaccaat gttctgcagc ctccagccaa cagacctcag 1920

gaaagagggg atgaacttgc agactctgcg cttgagatct tcaaacaagc atcagcgttt 1980

tccagggctt cccagaggtc tgtgcgacta gcccctgtct atcaaaagtt attagagagg 2040

atgaagcatt ag 2052

<210> 7
<211> 9293
<212> DNA
<213> Artificial Sequence

<220>
<223> pX458-hTGFBI(R124H)gRNA Plasmid

<400> 7
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacacca ctcagctgta cacggaccac agttttagag ctagaaatag caagttaaaa 300

taaggctagt ccgttatcaa cttgaaaaag tggcaccgag tcggtgcttt tttgttttag 360

agctagaaat agcaagttaa aataaggcta gtccgttttt agcgcgtgcg ccaattctgc 420

agacaaatgg ctctagaggt acccgttaca taacttacgg taaatggccc gcctggctga 480

ccgcccaacg acccccgccc attgacgtca ataatgacgt atgttcccat agtaacgcca 540

cgtcaatggg tggagtattt acggtaaact gcccacttgg cagtacatca agtgtatcat 600

atgccaagta cgccccctat tgacgtcaat gacggtaaat ggcccgcctg gcattgtgcc 660

cagtacatga ccttatggga ctttcctact tggcagtaca tctacgtatt agtcatcgct 720

attaccatgg tcgaggtgag ccccacgttc tgcttcactc tccccatctc ccccccctcc 780

ccacccccaa ttttgtattt atttattttt taattatttt gtgcagcgat gggggcgggg 840

gggggggggg ggcgcgcgcc aggcggggcg gggcggggcg aggggcgggg cggggcgagg 900

cggagaggtg cggcggcagc caatcagagc ggcgcgctcc gaaagtttcc ttttatggcg 960

aggcggcggc ggcggcggcc ctataaaaag cgaagcgcgc ggcgggcggg agtcgctgcg 1020

acgctgcctt cgccccgtgc cccgctccgc cgccgcctcg cgccgcccgc cccggctctg 1080

actgaccgcg ttactcccac aggtgagcgg gcgggacggc ccttctcctc cgggctgtaa 1140

ttagctgagc aagaggtaag ggtttaaggg atggttggtt ggtggggtat taatgtttaa 1200

ttacctggag cacctgcctg aaatcacttt ttttcaggtt ggaccggtgc caccatggac 1260

tataaggacc acgacggaga ctacaaggat catgatattg attacaaaga cgatgacgat 1320

aagatggccc caaagaagaa gcggaaggtc ggtatccacg gagtcccagc agccgacaag 1380

aagtacagca tcggcctgga catcggcacc aactctgtgg gctgggccgt gatcaccgac 1440

gagtacaagg tgcccagcaa gaaattcaag gtgctgggca acaccgaccg gcacagcatc 1500

aagaagaacc tgatcggagc cctgctgttc gacagcggcg aaacagccga ggccacccgg 1560

ctgaagagaa ccgccagaag aagatacacc agacggaaga accggatctg ctatctgcaa 1620

gagatcttca gcaacgagat ggccaaggtg gacgacagct tcttccacag actggaagag 1680

tccttcctgg tggaagagga taagaagcac gagcggcacc ccatcttcgg caacatcgtg 1740

gacgaggtgg cctaccacga gaagtacccc accatctacc acctgagaaa gaaactggtg 1800

gacagcaccg acaaggccga cctgcggctg atctatctgg ccctggccca catgatcaag 1860

ttccggggcc acttcctgat cgagggcgac ctgaaccccg acaacagcga cgtggacaag 1920

ctgttcatcc agctggtgca gacctacaac cagctgttcg aggaaaaccc catcaacgcc 1980

agcggcgtgg acgccaaggc catcctgtct gccagactga gcaagagcag acggctggaa 2040

aatctgatcg cccagctgcc cggcgagaag aagaatggcc tgttcggaaa cctgattgcc 2100

ctgagcctgg gcctgacccc caacttcaag agcaacttcg acctggccga ggatgccaaa 2160

ctgcagctga gcaaggacac ctacgacgac gacctggaca acctgctggc ccagatcggc 2220

gaccagtacg ccgacctgtt tctggccgcc aagaacctgt ccgacgccat cctgctgagc 2280

gacatcctga gagtgaacac cgagatcacc aaggcccccc tgagcgcctc tatgatcaag 2340

agatacgacg agcaccacca ggacctgacc ctgctgaaag ctctcgtgcg gcagcagctg 2400

cctgagaagt acaaagagat tttcttcgac cagagcaaga acggctacgc cggctacatt 2460

gacggcggag ccagccagga agagttctac aagttcatca agcccatcct ggaaaagatg 2520

gacggcaccg aggaactgct cgtgaagctg aacagagagg acctgctgcg gaagcagcgg 2580

accttcgaca acggcagcat cccccaccag atccacctgg gagagctgca cgccattctg 2640

cggcggcagg aagattttta cccattcctg aaggacaacc gggaaaagat cgagaagatc 2700

ctgaccttcc gcatccccta ctacgtgggc cctctggcca ggggaaacag cagattcgcc 2760

tggatgacca gaaagagcga ggaaaccatc accccctgga acttcgagga agtggtggac 2820

aagggcgctt ccgcccagag cttcatcgag cggatgacca acttcgataa gaacctgccc 2880

aacgagaagg tgctgcccaa gcacagcctg ctgtacgagt acttcaccgt gtataacgag 2940

ctgaccaaag tgaaatacgt gaccgaggga atgagaaagc ccgccttcct gagcggcgag 3000

cagaaaaagg ccatcgtgga cctgctgttc aagaccaacc ggaaagtgac cgtgaagcag 3060

ctgaaagagg actacttcaa gaaaatcgag tgcttcgact ccgtggaaat ctccggcgtg 3120

gaagatcggt tcaacgcctc cctgggcaca taccacgatc tgctgaaaat tatcaaggac 3180

aaggacttcc tggacaatga ggaaaacgag gacattctgg aagatatcgt gctgaccctg 3240

acactgtttg aggacagaga gatgatcgag gaacggctga aaacctatgc ccacctgttc 3300

gacgacaaag tgatgaagca gctgaagcgg cggagataca ccggctgggg caggctgagc 3360

cggaagctga tcaacggcat ccgggacaag cagtccggca agacaatcct ggatttcctg 3420

aagtccgacg gcttcgccaa cagaaacttc atgcagctga tccacgacga cagcctgacc 3480

tttaaagagg acatccagaa agcccaggtg tccggccagg gcgatagcct gcacgagcac 3540

attgccaatc tggccggcag ccccgccatt aagaagggca tcctgcagac agtgaaggtg 3600

gtggacgagc tcgtgaaagt gatgggccgg cacaagcccg agaacatcgt gatcgaaatg 3660

gccagagaga accagaccac ccagaaggga cagaagaaca gccgcgagag aatgaagcgg 3720

atcgaagagg gcatcaaaga gctgggcagc cagatcctga aagaacaccc cgtggaaaac 3780

acccagctgc agaacgagaa gctgtacctg tactacctgc agaatggcgg ggatatgtac 3840

gtggaccagg aactggacat caaccggctg tccgactacg atgtggacca tatcgtgcct 3900

cagagctttc tgaaggacga ctccatcgac aacaaggtgc tgaccagaag cgacaagaac 3960

cggggcaaga gcgacaacgt gccctccgaa gaggtcgtga agaagatgaa gaactactgg 4020

cggcagctgc tgaacgccaa gctgattacc cagagaaagt tcgacaatct gaccaaggcc 4080

gagagaggcg gcctgagcga actggataag gccggcttca tcaagagaca gctggtggaa 4140

acccggcaga tcacaaagca cgtggcacag atcctggact cccggatgaa cactaagtac 4200

gacgagaatg acaagctgat ccgggaagtg aaagtgatca ccctgaagtc caagctggtg 4260

tccgatttcc ggaaggattt ccagttttac aaagtgcgcg agatcaacaa ctaccaccac 4320

gcccacgacg cctacctgaa cgccgtcgtg ggaaccgccc tgatcaaaaa gtaccctaag 4380

ctggaaagcg agttcgtgta cggcgactac aaggtgtacg acgtgcggaa gatgatcgcc 4440

aagagcgagc aggaaatcgg caaggctacc gccaagtact tcttctacag caacatcatg 4500

aactttttca agaccgagat taccctggcc aacggcgaga tccggaagcg gcctctgatc 4560

gagacaaacg gcgaaaccgg ggagatcgtg tgggataagg gccgggattt tgccaccgtg 4620

cggaaagtgc tgagcatgcc ccaagtgaat atcgtgaaaa agaccgaggt gcagacaggc 4680

ggcttcagca aagagtctat cctgcccaag aggaacagcg ataagctgat cgccagaaag 4740

aaggactggg accctaagaa gtacggcggc ttcgacagcc ccaccgtggc ctattctgtg 4800

ctggtggtgg ccaaagtgga aaagggcaag tccaagaaac tgaagagtgt gaaagagctg 4860

ctggggatca ccatcatgga aagaagcagc ttcgagaaga atcccatcga ctttctggaa 4920

gccaagggct acaaagaagt gaaaaaggac ctgatcatca agctgcctaa gtactccctg 4980

ttcgagctgg aaaacggccg gaagagaatg ctggcctctg ccggcgaact gcagaaggga 5040

aacgaactgg ccctgccctc caaatatgtg aacttcctgt acctggccag ccactatgag 5100

aagctgaagg gctcccccga ggataatgag cagaaacagc tgtttgtgga acagcacaag 5160

cactacctgg acgagatcat cgagcagatc agcgagttct ccaagagagt gatcctggcc 5220

gacgctaatc tggacaaagt gctgtccgcc tacaacaagc accgggataa gcccatcaga 5280

gagcaggccg agaatatcat ccacctgttt accctgacca atctgggagc ccctgccgcc 5340

ttcaagtact ttgacaccac catcgaccgg aagaggtaca ccagcaccaa agaggtgctg 5400

gacgccaccc tgatccacca gagcatcacc ggcctgtacg agacacggat cgacctgtct 5460

cagctgggag gcgacaaaag gccggcggcc acgaaaaagg ccggccaggc aaaaaagaaa 5520

aaggaattcg gcagtggaga gggcagagga agtctgctaa catgcggtga cgtcgaggag 5580

aatcctggcc cagtgagcaa gggcgaggag ctgttcaccg gggtggtgcc catcctggtc 5640

gagctggacg gcgacgtaaa cggccacaag ttcagcgtgt ccggcgaggg cgagggcgat 5700

gccacctacg gcaagctgac cctgaagttc atctgcacca ccggcaagct gcccgtgccc 5760

tggcccaccc tcgtgaccac cctgacctac ggcgtgcagt gcttcagccg ctaccccgac 5820

cacatgaagc agcacgactt cttcaagtcc gccatgcccg aaggctacgt ccaggagcgc 5880
accatcttct tcaaggacga cggcaactac aagacccgcg ccgaggtgaa gttcgagggc 5940
gacaccctgg tgaaccgcat cgagctgaag ggcatcgact tcaaggagga cggcaacatc 6000
ctggggcaca agctggagta caactacaac agccacaacg tctatatcat ggccgacaag 6060
cagaagaacg gcatcaaggt gaacttcaag atccgccaca acatcgagga cggcagcgtg 6120
cagctcgccg accactacca gcagaacacc cccatcggcg acggccccgt gctgctgccc 6180
gacaaccact acctgagcac ccagtccgcc ctgagcaaag accccaacga gaagcgcgat 6240
cacatggtcc tgctggagtt cgtgaccgcc gccgggatca ctctcggcat ggacgagctg 6300
tacaaggaat tctaactaga gctcgctgat cagcctcgac tgtgccttct agttgccagc 6360
catctgttgt ttgcccctcc cccgtgcctt ccttgaccct ggaaggtgcc actcccactg 6420
tcctttccta ataaaatgag gaaattgcat cgcattgtct gagtaggtgt cattctattc 6480
tggggggtgg ggtggggcag gacagcaagg gggaggattg ggaagagaat agcaggcatg 6540
ctggggagcg gccgcaggaa cccctagtga tggagttggc cactccctct ctgcgcgctc 6600
gctcgctcac tgaggccggg cgaccaaagg tcgcccgacg cccgggcttt gcccgggcgg 6660
cctcagtgag cgagcgagcg cgcagctgcc tgcaggggcg cctgatgcgg tattttctcc 6720
ttacgcatct gtgcggtatt tcacaccgca tacgtcaaag caaccatagt acgcgccctg 6780
tagcggcgca ttaagcgcgg cgggtgtggt ggttacgcgc agcgtgaccg ctacacttgc 6840
cagcgcccta gcgcccgctc ctttcgcttt cttcccttcc tttctcgcca cgttcgccgg 6900
ctttccccgt caagctctaa atcgggggct ccctttaggg ttccgattta gtgctttacg 6960
gcacctcgac cccaaaaaac ttgatttggg tgatggttca cgtagtgggc catcgccctg 7020
atagacggtt tttcgccctt tgacgttgga gtccacgttc tttaatagtg gactcttgtt 7080
ccaaactgga acaacactca accctatctc gggctattct tttgatttat aagggatttt 7140
gccgatttcg gcctattggt taaaaaatga gctgatttaa caaaaattta acgcgaattt 7200
taacaaaata ttaacgttta caattttatg gtgcactctc agtacaatct gctctgatgc 7260
cgcatagtta agccagcccc gacacccgcc aacacccgct gacgcgccct gacgggcttg 7320
tctgctcccg gcatccgctt acagacaagc tgtgaccgtc tccgggagct gcatgtgtca 7380
gaggttttca ccgtcatcac cgaaacgcgc gagacgaaag ggcctcgtga tacgcctatt 7440
tttataggtt aatgtcatga taataatggt ttcttagacg tcaggtggca cttttcgggg 7500
aaatgtgcgc ggaaccccta tttgtttatt tttctaaata cattcaaata tgtatccgct 7560
catgagacaa taaccctgat aaatgcttca ataatattga aaaaggaaga gtatgagtat 7620
tcaacatttc cgtgtcgccc ttattccctt ttttgcggca ttttgccttc ctgtttttgc 7680

tcacccagaa acgctggtga aagtaaaaga tgctgaagat cagttgggtg cacgagtggg 7740

ttacatcgaa ctggatctca acagcggtaa gatccttgag agttttcgcc ccgaagaacg 7800

ttttccaatg atgagcactt ttaaagttct gctatgtggc gcggtattat cccgtattga 7860

cgccgggcaa gagcaactcg gtcgccgcat acactattct cagaatgact tggttgagta 7920

ctcaccagtc acagaaaagc atcttacgga tggcatgaca gtaagagaat tatgcagtgc 7980

tgccataacc atgagtgata acactgcggc caacttactt ctgacaacga tcggaggacc 8040

gaaggagcta accgcttttt tgcacaacat gggggatcat gtaactcgcc ttgatcgttg 8100

ggaaccggag ctgaatgaag ccataccaaa cgacgagcgt gacaccacga tgcctgtagc 8160

aatggcaaca acgttgcgca aactattaac tggcgaacta cttactctag cttcccggca 8220

acaattaata gactggatgg aggcggataa agttgcagga ccacttctgc gctcggccct 8280

tccggctggc tggtttattg ctgataaatc tggagccggt gagcgtggaa gccgcggtat 8340

cattgcagca ctggggccag atggtaagcc ctcccgtatc gtagttatct acacgacggg 8400

gagtcaggca actatggatg aacgaaatag acagatcgct gagataggtg cctcactgat 8460

taagcattgg taactgtcag accaagttta ctcatatata ctttagattg atttaaaact 8520

tcatttttaa tttaaaagga tctaggtgaa gatccttttt gataatctca tgaccaaaat 8580

cccttaacgt gagttttcgt tccactgagc gtcagacccc gtagaaaaga tcaaaggatc 8640

ttcttgagat cctttttttc tgcgcgtaat ctgctgcttg caaacaaaaa aaccaccgct 8700

accagcggtg gtttgtttgc cggatcaaga gctaccaact ctttttccga aggtaactgg 8760

cttcagcaga gcgcagatac caaatactgt ccttctagtg tagccgtagt taggccacca 8820

cttcaagaac tctgtagcac cgcctacata cctcgctctg ctaatcctgt taccagtggc 8880

tgctgccagt ggcgataagt cgtgtcttac cgggttggac tcaagacgat agttaccgga 8940

taaggcgcag cggtcgggct gaacgggggg ttcgtgcaca cagcccagct tggagcgaac 9000

gacctacacc gaactgagat acctacagcg tgagctatga gaaagcgcca cgcttcccga 9060

agggagaaag gcggacaggt atccggtaag cggcagggtc ggaacaggag agcgcacgag 9120

ggagcttcca gggggaaacg cctggtatct ttatagtcct gtcgggtttc gccacctctg 9180

acttgagcgt cgatttttgt gatgctcgtc aggggggcgg agcctatgga aaaacgccag 9240

caacgcggcc tttttacggt tcctggcctt ttgctggcct tttgctcaca tgt 9293

<210> 8
<211> 102
<212> RNA
<213> Artificial Sequence

<220>
<223> pX458-hTGFBI(R124H)gRNA

<400>  8
acucagcugu acacggacca caguuuuaga gcuagaaaua gcaaguuaaa auaaggcuag      60

uccguuauca acuugaaaaa guggcaccga gucggugcuu uu                        102


<210>  9
<211>  21
<212>  DNA
<213>  Artificial Sequence

<220>
<223>  Forward primer

<400>  9
gttgagttca cgtagacagg c                                                21


<210>  10
<211>  21
<212>  DNA
<213>  Artificial Sequence

<220>
<223>  Reverse primer

<400>  10
gactcccatt catcatgccc a                                                21


<210>  11
<211>  20
<212>  DNA
<213>  Artificial Sequence

<220>
<223>  OTS1 primer

<400>  11
atgtcagaag tcccgctgtg                                                  20


<210>  12
<211>  20
<212>  DNA
<213>  Artificial Sequence

<220>
<223>  OTS1 primer

<400>  12
tgatggggtc agagggcata                                                  20


<210>  13
<211>  22
<212>  DNA
<213>  Artificial Sequence

<220>
<223>  OTS2 primer

<400>  13

cttcctgctc tgtgtttagc ca                                                22

<210>  14
<211>  21
<212>  DNA
<213>  Artificial Sequence

<220>
<223>  OTS2 primer

<400>  14
acctccaagt tgagcagtgt c                                                 21

<210>  15
<211>  20
<212>  DNA
<213>  Artificial Sequence

<220>
<223>  OTS3 primer

<400>  15
gcagcaaagc actcaagagg                                                   20

<210>  16
<211>  20
<212>  DNA
<213>  Artificial Sequence

<220>
<223>  OTS3 primer

<400>  16
caaacttctg cctgggcatc                                                   20